(19) Europäisches Patentamt
European Patent Office
Office européen des brevets

(11) Veröffentlichungsnummer: **0 246 195**
**B1**

(12) # EUROPÄISCHE PATENTSCHRIFT

(45) Veröffentlichungstag der Patentschrift: **13.12.89**

(51) Int. Cl.⁴: **C07F 15/00**, C07C 29/136

(21) Anmeldenummer: **87810295.3**

(22) Anmeldetag: **11.05.87**

(54) **Optisch aktive Iridiumkomplexe und deren Verwendung.**

(30) Priorität: **16.05.86 CH 1987/86**

(43) Veröffentlichungstag der Anmeldung: **19.11.87 Patentblatt 87/47**

(45) Bekanntmachung des Hinweises auf die Patenterteilung: **13.12.89 Patentblatt 89/50**

(84) Benannte Vertragsstaaten: **AT BE CH DE FR GB IT LI NL SE**

(56) Entgegenhaltungen:
**JOURNAL OF ORGANOMETALLIC CHEMISTRY, Band 222, 1981, Seiten 323-329, Elsevier Sequoia S.A., Lausanne, CH; G. ZASSINOVICH et al.: "Enantioselective transfer hydrogenation catalyzed by irridium(I) complexes with nitrogen donor ligands"**

(73) Patentinhaber: **CIBA-GEIGY AG, Klybeckstrasse 141, CH-4002 Basel(CH)**

(72) Erfinder: **Kaschig, Jürgen, Dr., Rötebuckweg 30, D-7800 Freiburg(DE)**

**EP 0 246 195 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft optisch aktive kationische Iridium(I)-komplexe mit asymmetrischen Pyridinaldiminliganden sowie Dienliganden, ein Verfahren zu deren Herstellung und deren Verwendung als enantioselektive Katalysatoren.

G. Zassinovich et al. beschreiben im Journal of Organometallic Chemistry, 222, S.323-329 (1981) kationische Iridium(I)-komplexe mit einem 1,5-Cycloactadienliganden und einem 2-Pyridinaldiminliganden, der am Imin-N-Atom durch optische aktives α-Phenylethyl oder 3-Pinanmethyl substituiert ist. Sie wirken als enantioselektive homogene Katalysatoren bei der Transferhydrierung von prochiralen Ketonen mit Isopropanol. Bei der Reaktion werden zwar hohe Ausbeuten erzielt, aber die optische Ausbeute (Enantiomerenüberschuss) ist relativ gering.

Es wurde nun gefunden, das die optische Ausbeute wesentlich erhöht werden kann, wenn in den erwähnten Iridiumkomplexen im Pyridinaldiminliganden in 6-Stellung eine Methylgruppe oder am Imin-N-Atom dieses Liganden ein cyclischer Rest mit mindestens zwei chiralen C-Atomen und einer Alkyl- oder Phenylgruppe in α-Stellung gebunden ist.

Gegenstand der Erfindung sind optisch aktive Iridiumkomplexe der Formel (I)

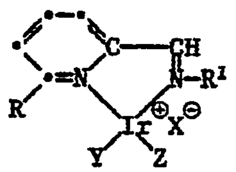

(I),

worin

R Methyl und $R^1$ ein Kohlenwasserstoffrest mit mindestens einem chiralen C-Atom oder mindestens ein Heteroatom enthaltender Kohlenwasserstoffrest mit mindestens einen chiralen C-Atom bedeuten; oder R ein Wasserstoffatom und $R^1$ 1,2-Diphenylethyl oder ein 5- oder 6-gliedriger cycloaliphatischer oder cycloheteroaliphatischer Rest mit mindestens zwei chiralen C-Atomen und einer Phenylgruppe oder Alkylgruppe in α-Stellung bedeuten,

$X^\ominus$ das Anion einer einbasischen anorganischen oder organischen Säure ist, und

Y und Z je Ethylen oder Y und Z zusammen ein offenkettiges oder cyclisches Dien mit 6 bis 10 C-Atomen, dessen Diengruppen über 1 oder 2 C-Atome verbunden sind, bedeuten.

Optisch aktiv bedeutet, dass mindestens ein chirales C-Atom überwieged R- oder S-Konfiguration aufweist. Die Iridiumkomplexe der Formel I umfassen auch Gemische von Diastereoisomeren.

Bei $X^\ominus$ als Anion einer einbasischen anorganischen oder organischen Säure kann es sich zum Beispiel um $F^\ominus$, $Cl^\ominus$, $Br^\ominus$, $J^\ominus$, $ClO_4^\ominus$, $NO_3^\ominus$, $BrO_3^\ominus$, $HSO_4^\ominus$, $H_2PO_3^\ominus$, $BF_4^\ominus$, $PF_6^\ominus$, $SbF_6^\ominus$, $AsF_6^\ominus$, $SbCl_6^\ominus$, $SbCl_5F^\ominus$, $HCOO^\ominus$, $CH_3COO^\ominus$, $CCl_3COO^\ominus$, $CF_3COO^\ominus$, $CH_3SO_3^\ominus$, $CCl_3SO_3^\ominus$, $CF_3SO_3^\ominus$, Phenyl-$SO_3^\ominus$ oder p-Toluyl-$SO_3^\ominus$ handeln. In einer bevorzugten Ausführungsform stellt $X^\ominus$ $BF_4^\ominus$, $ClO_4^\ominus$, $CF_3SO_3^\ominus$, $PF_6^\ominus$ oder Halogenid dar. Unter den Halogeniden sind Chlorid, Bromid und Jodid bevorzugt.

Y und Z stehen bevorzugt je für Ethylen oder Y und Z stellen zusammen bevorzugt ein Dien mit 6 bis 8 C-Atomen, dessen Diengruppen insbesondere über 2 C-Atome verbunden sind, dar. In einer bevorzugten Ausführungsform stehen Y und Z je für Ethylen oder Y und Z zusammen für 1,5-Cycloactadien, Norbornadien oder 1,5-Hexadien.

Eine bevorzugte Untergruppe der Iridiumkomplexe der Formel (I), sind solche, worin R für Methyl steht und $R^1$ ein Rest der Formel II

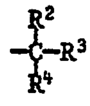

(II)

ist, worin

$R^2$, $R^3$ und $R^4$ voneinander verschieden sind, wenn sie nicht mindestens 1 chirales C-Atom enthalten, und für ein Wasserstoffatom, $C_1$-$C_4$-Alkyl, $C_1$-$C_4$-Alkoxy, unsubstituiertes oder durch $C_1$-$C_4$-Alkyl oder Phenyl substituiertes Cycloalkyl mit 5 bis 7 Ring-C-Atomen, unsubstituiertes oder durch $C_1$-$C_4$-Alkyl oder Phenyl substituiertes Cycloalkylalkyl mit 5 bis 7 Ring-C-Atomen und 1 oder 2 C-Atomen in der Alkylengruppe, Phenyl, Naphthyl, Benzyl oder β-Phenylethyl sind oder $R^3$ und $R^4$ zusammen durch $C_1$-$C_4$-Alkyl oder Phenyl substituiertes lineares $C_4$-oder $C_5$-Alkylen, $C_3$-oder $C_4$-Oxaalkylen oder $C_3$-Dioxaalkylen mit mindestens einem chiralen C-Atom bedeuten.

Bei den $C_1$-$C_4$-Alkyl- und $C_1$-$C_4$-Alkoxyresten kann es sich um Methyl, Ethyl, n-Propyl, i-Propyl, n-Butyl, i-Butyl, t-Butyl und die entsprechenden Alkoxyreste handeln. $R^2$, $R^3$ und $R^4$ sind als Alkyl bevorzugt Methyl oder Ethyl und als Alkoxy bevorzugt Methoxy. $R^2$ bis $R^4$ bedeuten als Cycloalkyl bevorzugt

Cyclopentyl, Cycloheptyl und besonders Cyclohexyl. Bei $R^3$ bis $R^4$ als Cycloalkylalkyl handelt es sich bevorzugt um (Cyclohexyl)methyl. $R^3$ und $R^4$ zusammen bedeuten als $C_3$- oder $C_4$- Oxaalkylen bevorzugt 2-Oxapentylen und als $C_3$-Dioxaalkylen bevorzugt 2,4-Dioxapentylen. $C_1$-$C_4$-Alkyl als Substituent für $R^2$ bis $R^4$ kann Methyl, Ethyl, n-Propyl, i-Propyl und Butyl sein. Bevorzugte Substituenten sind Methyl und Phenyl.

$R^2$ in Formel (II) ist bevorzugt H. Eine bevorzugte Untergruppe sind solche Iridiumkomplexe der Formel (I), worin $R^2$ H bedeutet, $R^3$ für $C_1$-$C_4$-Alkyl, $C_1$-$C_4$-Alkoxy oder Phenyl und $R_4$ für Phenyl, Benzyl oder Naphthyl stehen, wobei $R_3$ und $R_4$ nicht beide Phenyl sind; oder $R^2$ und $R^4$ je H bedeuten und $R^3$ der Formel

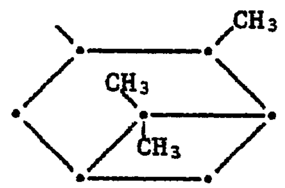

entspricht; oder die Gruppe-$CR^2R^3R^4$ der Formel

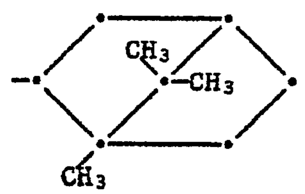

entspricht; oder $R^2$ für H steht und $R^3$ und $R^4$ zusammen in 2-Stellung durch $C_1$-$C_4$-Alkyl substituiertes Pentamethylen oder in 1- und/oder 3-Stellung durch $C_1$-$C_4$-Alkyl oder Phenyl substituiertes 2,4-Dioxapentylen bedeuten.

Eine weitere bevorzugte Ausführungsform sind Iridiumkomplexe der Formel (I), worin R für ein Wasserstoffatom steht und $R^1$ 1,2-Diphenylethyl ist, oder für einen Rest der Formeln

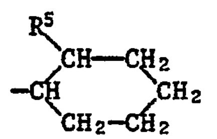

oder

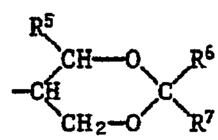

steht, worin $R^5$ $C_1$-$C_4$-Alkyl, Benzyl oder Phenyl bedeuten und $R^6$ und $R^7$ unabhängig von einander für H, $C_1$-$C_4$-Alkyl oder Phenyl stehen. $R^5$ ist bevorzugt Phenyl oder Methyl und $R^6$ und $R^7$ sind bevorzugt H, Methyl oder Phenyl. Besonders bevorzugt ist $R^5$ Phenyl und $R^6$ und $R^7$ Methyl oder $R^6$ H und $R^7$ Phenyl.

Die Iridiumkomplexe der Formel (I) können nach an sich bekannten Verfahren [siehe J. of Organom. Chem., 222, S. 323-329 (1981)] erhalten werden, indem man einen Diiridiumkomplex der Formel $[Ir(YZ)Cl]_2$ mit einem Pyridinalimin der Formel (III)

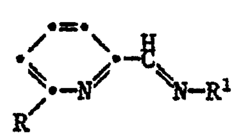

(III)

umsetzt, wobei R, $R^1$, Y und Z die oben angegebene Bedeutung haben, und anschliessend das gebildete Chlorid der Formel (I) zur Herstellung anderer Salze mit einem Salz $M^{\oplus}X'^{\ominus}$, worin M für ein Alkalimetall steht und $X'^{\ominus}$ die Bedeutung von $X^{\ominus}$ - ausgenommen Chlorid - hat, umsetzt.

Die Diiridiumkomplexe sind bekannt oder können nach bekannten Verfahren hergestellt werden, indem man ein Dichlorotetrakis(alken)diiridium (I) (Alken zum Beispiel Cycloocten) mit Ethylen oder einem Dien YZ umsetzt.

Die Umsetzungen werden im allgemeinen bei Temperaturen von -10 bis 30°C in einem inerten Lösungsmittel und unter Luftausschluss durchgeführt. Geeignete inerte Lösungsmittel sind z.B. Kohlenwasserstoffe wie Benzol, Toluol, Xylol, Petrolether, Hexan, Cyclohexan, Methylcyclohexan; und Ether wie z.B. Diethylether, Dibutylether, Tetrahydrofuran und Dioxan.

Bei der Reaktion erhält man zunächst die Chloride der Formel (I). Zur Herstellung der Salze der Formel (I) mit anderen Anionen einbasischer anorganischer oder organischer Säuren können die Chloride

der Formel (I) entweder direkt nach der Reaktion oder nach der Isolierung und Reinigung und dem erneuten Lösen in polaren Lösungsmitteln (z.B. Alkoholen, Ethern oder Ketonen, gegebenenfalls unter Zusatz von Wasser) mit einem Alkalisalz $M^{\oplus}X'^{\ominus}$ umgesetzt und danach isoliert werden. $X'^{\ominus}$ steht für ein von $X^{\ominus}$ verschiedenes Anion. $M^{\oplus}$ steht bevorzugt für Natrium. Die erfindungsgemässen Iridiumkomplexe sind kristallin und können durch Filtration isoliert und durch Umkristallisation gereinigt werden.

Die Pyridinaldimine der Formel (III) sind bekannt oder können in an sich bekannter Weise durch die Umsetzung des gegebenenfalls durch Methyl substituierten 2-Pyridinaldehyds mit einem Amin $R^1NH_2$ erhalten werden. Zweckmässig werden reine Stereoisomere der Amine $R^1NH_2$ verwendet, so dass man direkt zu reinen Enantiomeren der Formel (III) gelangt. Man kann aber auch Enantiomerengemische einsetzen und anschliessend die gebildete Racemate Enantiomerengemische der Formel (III) nach klassischen Methoden auftrennen.

Stereoisomere der Amine $R^1NH_2$ sind bekannt, teilweise käuflich oder nach bekannten Verfahren herstellbar. Beispiele für solche Amine sind:
(R)-2-Aminobutan, (R)-1-Phenylethylamin, (S)-1-Phenylethylamin, (R)-1-(α-Naphthyl)ethylamin, (S)-2-Amino-3-phenylpropan, (R)-1,2-Diphenylethylamin, (S)-Alaninol, (S)-Phenylalaninol, (4S, 5S)-5-Amino-2,2-dimethyl-4-phenyl-1,3-dioxan, (S)-2-Amino-1-methoxy-3-phenylpropan, (R)-Bornylamin, (R)-3-Aminomethylpinan, (+)-Dehydroabiethylamin, (2R, 4S, 5S)-(+)-5-Amino-2,4-Diphenyl-1,3-dioxan [Chem. Ber. 113, S. 710-721 (1980)], (1S, 2R)-(-)-2-Methylcyclohexylamin [Chem. Ber. 117, S.2076-2098 (1984)], (1S, 2S)-(+)-2-Phenylcyclohexylamin [Chem. Ber. 117, S. 2076-2098 (1984)].

Ein weiterer Gegenstand der Erfindung ist die Verwendung der erfindungsgemässen Iridiumkomplexe als enantioselektive homogene Katalysatoren für die Transferhydrierung insbesondere prochiraler Ketone mit sekundären Alkoholen. Als sekundärer Alkohol ist besonders Isopropanol geeignet. Die Reaktion wird vorteilhaft unter Sauerstoffausschluss bei erhöhter Temperatur durchgeführt. Zweckmässig wird der verwendete sekundäre Alkohol als Lösungsmittel verwendet. Die Katalysatormenge beträgt bevorzugt $10^{-2}$ bis $10^{-5}$ Mol/l, bezogen auf das Reaktionsvolumen. Die Reaktion wird bevorzugt in Gegenwart einer Base, besonders NaOH, durchgeführt.

Die nachfolgenden Beispiele erläutern die Erfindung näher. Die Bestimmung des Enantiomerenüberschusses (ee) erfolgt nach Mosher [J. Org. Chem. 34, S. 2543 (1969)].

<u>Beispiele 1-10</u>: Unter Argonschutzgas werden 0.2 g (0.226 mmol) Di-μ-chlorotetrakis(cycloocten)diiridium(I) in 18 ml Benzol gelöst. In den Beispielen 1,3 sowie 5-10 werden bei 10°C 1,56 ml 1,5-Hexadien zugegeben; in den Beispielen 2 und 4 bei 5°C während 15 Minuten Ethylen eingeleitet.

Nach 30 Minuten rühren werden 0,5 mmol der gemäss Tabelle 1 substituierten 6-Methyl-pyridin-2-aldehyd-alkylimine (N,N-Ligand) zugegeben. Nach 1 Stunde Rühren bei Raumtemperatur werden unter Argonschutzgas (in den Beispielen 2 und 4 unter Ethylenatmosphäre) 40 ml n-Hexan zugegeben. Bei 0°C und im Verlauf von ca. 2 Stunden kristallisiert das gewünschte Produkt aus.

In den Beispielen 1, 3 sowie 5-10 wird es unter Argon abgesaugt, mit n-Hexan gewaschen und ca. 16 Stunden bei 0.1 Pa getrocknet. In den Beispielen 2 und 4 wird das Produkt unter Ethylengas abgesaugt, mit Ethylengas gesättigtem n-Hexan gewaschen und 30 Minuten bei ca. 1kPa getrocknet.

Farbe sowie Elementarzusammensetzung der erhaltenen Komplexe sind aus Tabelle 1 zu entnehmen.

Tabelle 1

| Bsp. Nr. | Komplex (HD=1,5-Hexadien) | N,N ist 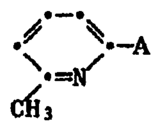 A (absolute Konfiguration) | Farbe | Elementaranalyse [Gew.-%] |
|---|---|---|---|---|
| 1 | [Ir(N,N)(HD)Cl] | 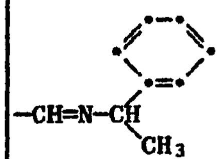 (S) | ziegelrot | Gef: C 47,68; H 5,04; N 5,41; Cl 6,63<br>Ber: C 47,22; H 4,91; N 5,25; Cl 6,64 |
| 2 | [Ir(N,N)($CH_2CH_2$)$_2$Cl] | 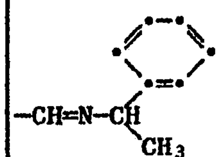 (S) | ziegelrot | Gef: C 44,99; H 4,50; N 5,42; Ir 36,90<br>Ber: C 44,92; H 4,76; N 5,51; Ir 37,83 |
| 3 | [Ir(N,N)(HD)Cl] | 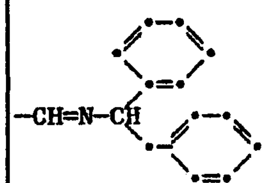 (R) | braun | Gef: C 53,15; H 4,96; N 4,59; Ir 31,50<br>Ber: C 53,22; H 5,18; N 4,17; Ir 31,40 |

Tabelle 1 (1. Fortsetzung)

| Bsp. Nr. | Komplex (HD=1,5-Hexadien) | N,N ist 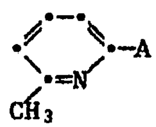 A (absolute Konfiguration) | Farbe | Elementaranalyse [Gew.-%] |
|---|---|---|---|---|
| 4 | [Ir(N,N)($CH_2CH_2$)$_2$Cl] | (R) 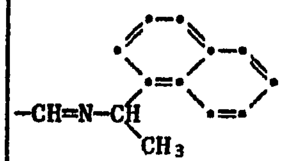 | bordeaux-rot | Gef: C 50,41; H 4,92; N 4,87; Ir 33,90<br>Ber: C 49,50; H 4,70; N 5,02; Ir 34,4 |
| 5 | [Ir(N,N)(HD)Cl] | (S) 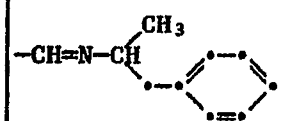 | ziegelrot | Gef: C 47,51; H 5,27; N 5,05; Cl 6,53<br>Ber: C 48,21; H 5,15; N 5,11; Cl 6,47 |
| 6 | [Ir(N,N)(HD)Cl] | (S) 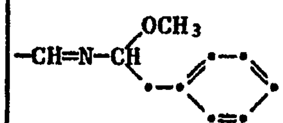 | beige | Gef: C 47,45; H 5,35; N 4.62; Ir 31,70<br>Ber: C 47,78; H 5,23; N 4,85; Ir 33,25 |
| 7 | [Ir(N,N)(HD)Cl] | (1S, 2S, 3S, 5R) 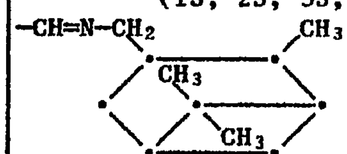 | hell-rot | Gef: C 49,42; H 6,39; N 4,69; Ir 31,30<br>Ber: C 49,68; H 6,25; N 4,83; Ir 33,13 |

**Tabelle 1** (2. Fortsetzung)

| Bsp. Nr. | Komplex (HD=1,5-Hexadien) | N,N ist 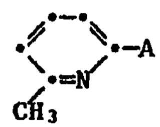 A (absolute Konfiguration) | Farbe | Elementaranalyse [Gew.-%] |
|---|---|---|---|---|
| 8 | [Ir(N,N)(HD)Cl] | 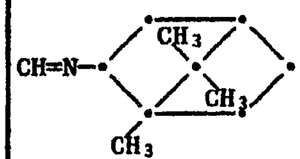 (1R) | braun-beige | Gef: C 47,27; H 6,04; N 4,81;<br>Ber: C 48,79; H 6,05; N 4,95 |
| 9 | [Ir(N,N)(HD)Cl] | 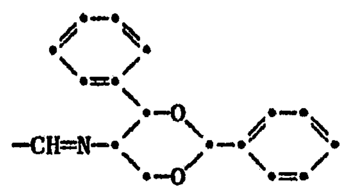 (2R, 4S, 5S) | hell-braun | Gef: C 52,29; H 4,87; N 3,90; Cl 5,25<br>Ber: C 52,12; H 4,83; N 4,19; Cl 5,31 |
| 10 | [Ir(N,N)(HD)Cl] | 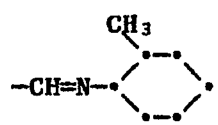 (1S, 2R) | ziegelrot | Gef: C 45,42; H 5,87; N 5,12; Cl 6,80<br>Ber: C 45,66; H 5,75; N 5,32; Cl 6,74 |

Beispiel 11: 214 mg (0,4 mmol) des gemäss Beispiel 1 erhaltenen Chlorokomplexes wird unter Argonschutzgas bei 40°C in einer Mischung aus 32 ml Aceton und 8 ml Wasser gelöst. Bei 25°C werden im Abstand von 30 Minuten zweimal je 150 mg Natriumjodid zugegeben. Bei 0°C kristallisiert ein hellbraunes Produkt aus. Es wird unter Argon abgesaugt, gründlich mit Wasser gewaschen und 20 Stunden bei 1 Pa über Phosphorpentoxid getrocknet.
Ausbeute: 120 mg

| Elementaranalyse | | | | | |
|---|---|---|---|---|---|
| Gef: | C 40,07; | H 4,20; | N 4,20; | J 19,61; | Ir 30,30% |
| Ber: | C 40,32; | H 4,14; | N 4,48; | J 20,29; | Ir 30,73% |

Beispiel 12: Analog Beispiel 11 werden 534 mg (0,8 mmol) des gemäss Beispiel 9 erhaltenen Chlorokomplexes mit Natriumjodid zur Reaktion gebracht. Es werden 150 mg dunkel-olivgrüne Kristalle erhalten.

| Elementaranalyse | | | | | |
|---|---|---|---|---|---|
| Gef: | C 45,20; | H 4,32; | N 3,45; | J 16,26; | Ir 25,30% |
| Ber: | C 45,85; | H 4,25; | N 3,69; | J 16,70; | Ir 25,30% |

Beispiele 13-17: Analog der Beispiele 1-10 werden die in Tabelle 2 beschriebenen substituierten Pyridin-2-aldehyd-alkylimine (N,N-Ligand) mit Di-µ-chlorotetrakis(cyclooocten)diiridium(I) sowie 1,5-Hexadien zur Reaktion gebracht. Nach analoger Aufarbeitung werden die in Tabelle 2 aufgeführten Komplexe erhalten.

Beispiel 18: Analog Beispiel 11 werden 523 mg (0,8 mmol) des gemäss Beispiel 13 erhaltenen Chlorokomplexes mit Natriumjodid zur Reaktion gebracht. Es werden 450 mg dunkelviolettes Produkt erhalten.

| Elementaranalyse | | | | | |
|---|---|---|---|---|---|
| Gef: | C 44,66; | H 4,34; | N 3,34; | J 16,84; | Ir 25,20% |
| Ber: | C 45,10; | H 4,06; | N 3,76; | J 17,02; | Ir 25,78%. |

Beispiel 19: Herstellung von

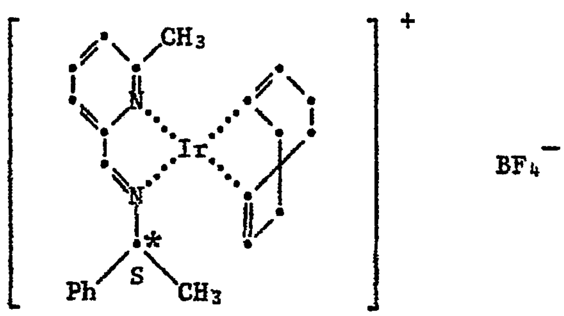

Unter Argon-Schutzgas werden 0,469 g (1,0 mMol) Bis(acetonitril) (cycloocta-1,5-dien)iridiumtetrafluoroborat in 15 ml Dichlormethan gelöst. Bei Raumtemperatur wird eine Lösung aus 5 ml Dichlormethan und 1,0 mMol 6-Methyl-pyridin-2-[N-α-phenylethylaldimin] zugegeben. Nach 1 Stunde wird das Reaktionsgemisch bei ca. 100 Pa bis zur Trockne eingeengt. Das ölige Gemisch wird mehrmals mit Diethylether gewaschen bis es fest wird. Es wird in 70 ml Methylenchlorid gelöst, mit 100 ml Diethylether als fester gelber Niederschlag gefällt, abfiltriert und 16 Stunden bei 0,1 Pa getrocknet.
Farbe: gelb
Mikroanalyse ($C_{23}H_{28}N_2BF_4Ir$):

| | C | H | N | F |
|---|---|---|---|---|
| Berechnet: | 45,18 | 4,62 | 4,58 | 12,43 |
| Gefunden: | 42,89 | 4,64 | 4,56 | 11,27 |

Tabelle 2

| Bsp. Nr. | Komplex (HD=1,5-Hexadien) | N,N ist 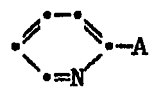 A (absolute Konfiguration) | Farbe | Elementaranalyse [Gew.-%] |
|---|---|---|---|---|
| 13 | [Ir(N,N)(HD)Cl] | 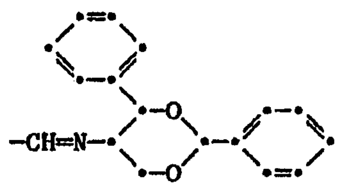 (2R, 4S, 5S) | dunkel-blau | Gef: C 51,54; H 4,68; N 4,11; Cl 5,38<br>Ber: C 51,41; H 4,62; N 4,28; Cl 5,42 |
| 14 | [Ir(N,N)(HD)Cl] | 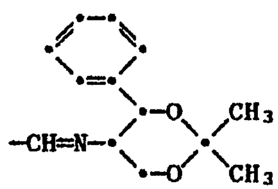 (2R, 4S, 5S) | dunkel-blau | Gef: C 46,13; H 4,95; N 4,15; Ir 31,00<br>Ber: C 47,55; H 4,99; N 4,62; Ir 31,71 |
| 15 | [Ir(N,N)(HD)Cl] | (1S, 2S) 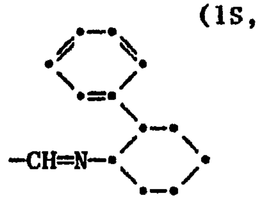 | stahl-blau | Gef: C 50,47; H 4,97; N 4,68<br>Ber: C 50,20; H 5,27; N 4,88 |

**Tabelle 2** (Fortsetzung)

| Bsp. Nr. | Komplex (HD=1,5-Hexadien) | N,N ist 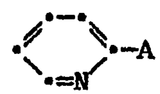 A (absolute Konfiguration) | Farbe | Elementaranalyse [Gew.-%] |
|---|---|---|---|---|
| 16 | [Ir(N,N)(HD)Cl] | 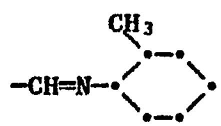 (1S, 2R) | violett | Gef: C 44,31; H 5,60; N 5,49; Cl 6,86<br>Ber: C 44,56; H 5,51; N 5,47; Cl 6,92 |
| 17 | [Ir(N,N)(HD)Cl] | 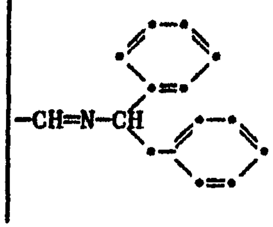 (R) | dunkel-blau | Gef:C 50,17;H 4,79;N 4,53;Cl 6,01;Ir 32,3<br>Ber:C 52,38;H 4,73;N 4,7;Cl 5,95;Ir 32,24 |

Beispiel 20 (Anwendungsbeispiel): 4,14 mg des gemäss Beispiel 1 hergestellten Komplexes werden unter Sauerstoffausschluss (Argonatmosphäre) in 36 ml Isopropanol gelöst. Nach 1 Stunde Rühren bei 60°C werden 0,38 ml 0,1 normale Natriumhydroxid-Lösung zugegeben. Es wird eine weitere Stunde bei 60°C gerührt und anschliessend eine Lösung aus 36 ml Isopropanol und 1,43 g Butyrophenon unter Sauerstoffausschluss hinzugefügt. Das molare Verhältnis Substrat zu Katalysator beträgt somit [S]/[Kat] = 1000, die Katalysatorkonzentration 1,33•10-4 Mol/l. Nach 21 Stunden bei 60°C wird gaschromatographisch (OV 101, 120°C, isotherm) eine Ausbeute an 1-Phenyl-1-butanol von 69,7 Gew.-% bestimmt.

Zur Bestimmung des Enantiomerengehalts nach Mosher wird eine Probe des Reaktionsgemisches (ca. 0,5 ml) weitgehend vom Lösungsmittel befreit und bei 0°C mit 50 ml optisch reinem α-Methoxy-α-trifluormethylphenyl-acetylchlorid sowie 0,25 ml trockenem Pryridin versetzt. Nach 15 Minuten wird 30 Minuten auf 70°C erwärmt, nach Abkühlen mit 3 ml 10%iger Citronensäurelösung versetzt und mit Ether die diastereomeren Ester extrahiert.

Gaschromatographisch (Kapilarsäule CW 20, 190°C) wird ein Enantiomerenüberschuss an (S)-1-Phenyl-1-butanol von ee=49,2 % bestimmt.

Beispiel 21: (Anwendungsbeispiel) Analog Beispiel 20 wird der Komplex nach Beispiel 18 zur katalytischen, enantioselektiven Transferhydrierung von Butyrophenon verwendet.

Nach 25 Stunden beträgt die Ausbeute an 1-Phenyl-1-butanol 68,6 Gew.-%, der Enantiomerenüberschuss ee beträgt 64,2 % (S).

**Patentansprüche für die Vertragsstaaten: BE, CH, DE, FR, GB, IT, LI, NL, SE**

1. Optisch aktive Iridiumkomplexe der Formel (I)

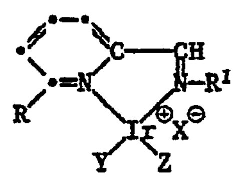

(I),

worin

R Methyl und $R^1$ ein Kohlenwasserstoffrest mit mindestens einem chiralen C-Atom oder mindestens ein Heteroatom enthaltender Kohlenwasserstoffrest mit mindestens einem chiralen C-Atom bedeuten; oder R ein Wasserstoffatom und $R^1$ 1,2-Diphenylethyl oder ein 5- oder 6-gliedriger cycloaliphatischer oder cycloheteroaliphatischer Rest mit mindestens zwei chiralen C-Atomen und einer Phenylgruppe oder Alkylgruppe in α-Stellung bedeuten,

$X^{\ominus}$ das Anion einer einbasischen anorganischen oder organischen Säure ist, und

Y und Z, je Ethylen oder Y und Z zusammen ein offenkettiges oder cyclisches Dien mit 6 bis 10 C-Atomen, dessen Diengruppen über 1 oder 2 C-Atome verbunden sind, bedeuten.

2. Iridiumkomplexe der Formel (I) gemäss Anspruch 1, worin $X^{\ominus}$ für Halogenid, $BF_4^{\ominus}$, $ClO_4^{\ominus}$, $CF_3SO_3^{\ominus}$ oder $PF_6^{\ominus}$ steht.

3. Iridiumkomplexe gemäss Anspruch 2, worin $X^{\ominus}$ als Halogenid, Chlorid, Bromid oder Jodid ist.

4. Iridiumkomplexe der Formel (I) gemäss Anspruch 1, worin Y und Z je für Ethylen oder Y und Z zusammen für 1,5-Cycloactadien, Norbornadien oder 1,5-Hexadien stehen.

5. Iridiumkomplexe der Formel (I) gemäss Anspruch 1, worin R für Methyl steht und $R^1$ ein Rest der Formel (II)

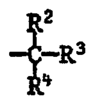

(II)

ist, worin $R^2$, $R^3$ und $R^4$ voneinander verschieden sind, wenn sie nicht mindestens 1 chirales C-Atom enthalten, und für ein Wasserstoffatom, $C_1$-$C_4$-Alkyl, $C_1$-$C_4$-Alkoxy, unsubstituiertes oder durch $C_1$-$C_4$-Alkyl oder Phenyl substituiertes Cycloalkyl mit 5 bis 7 Ring-C-Atomen, unsubstituiertes oder durch $C_1$-$C_4$-Alkyl oder Phenyl substituiertes Cycloalkylalkyl mit 5 bis 7 Ring-C-Atomen und 1 oder 2 C-Atomen in der Alkylengruppe, Phenyl, Naphthyl, Benzyl oder β-Phenylethyl sind oder $R^3$ und $R^4$ zusammen durch $C_1$-$C_4$-Alkyl oder Phenyl substituiertes lineares $C_4$-oder $C_5$-Alkylen, $C_3$-oder $C_4$-Oxaalkylen oder $C_3$-Dioxaalkylen mit mindestens einem chiralen C-Atom bedeuten.

6. Iridiumkomplexe der Formel (I) gemäss Anspruch 5, worin $R^2$ H bedeutet.

7. Iridiumkomplexe der Formel (I) gemäss Anspruch 5, worin $R^2$ H bedeutet, $R^3$ für $C_1$-$C_4$-Alkyl, $C_1$-$C_4$-Alkoxy oder Phenyl und $R_4$ für Phenyl, Benzyl oder Naphthyl stehen, wobei $R_3$ und $R_4$ nicht beide Phenyl sind; oder $R^2$ und $R^4$ je H bedeuten und $R^3$ der Formel

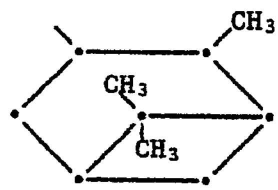

entspricht; oder die Gruppe $-CR^2R^3R^4$ der Formel

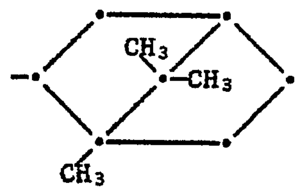

entspricht; oder $R^2$ für H steht und $R^3$ und $R^4$ zusammen in 2-Stellung durch $C_1$-$C_4$-Alkyl substituiertes Pentamethylen oder in 1- und/oder 3-Stellung durch $C_1$-$C_4$-Alkyl oder Phenyl substituiertes 2,4-Dioxapentylen bedeuten.

8. Iridiumkomplexe der Formel (I) gemäss Anspruch 1, worin R für ein Wasserstoffatom steht und $R^1$ 1,2-Diphenylethyl ist oder für einen Rest der Formeln

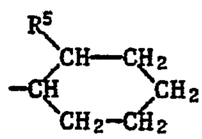

oder

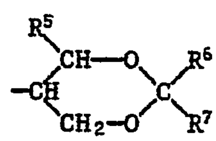

steht, worin $R^5$ $C_1$-$C_4$-Alkyl, Benzyl oder Phenyl bedeuten und $R^6$ und $R^7$ unabhängig voneinander für H, $C_1$-$C_4$-Alkyl oder Phenyl stehen.

9. Iridiumkomplexe der Formel (I) gemäss Anspruch 8, worin $R^5$ Phenyl und $R^6$ und $R^7$ Methyl oder $R^6$ H und $R^7$ Phenyl bedeuten.

10. Verwendung von optisch aktiven Iridiumkomplexen der Formel (I) gemäss Anspruch 1 als enantioselektive homogene Katalysatoren für die Transferhydrierung von prochiralen Ketonen mit sekundären Alkoholen.

11. Verfahren zur Herstellung von optisch aktiven Iridiumkomplexen der Formel (I) gemäss Anspruch 1, dadurch gekennzeichnet, dass man einen Diiridiumkomplex der Formel $[Ir(YZ)Cl]_2$ mit einem Pyridinalimin der Formel (III)

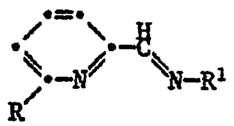

(III)

umsetzt, wobei R, $R^1$, Y und Z die in Anspruch 1 angegebene Bedeutung haben, und anschliessend das gebildete Chlorid der Formel (I) zur Herstellung anderer Salze mit einem Salz $M^{\oplus}X'^{\ominus}$, worin $M^{\oplus}$ für ein Alkalimetall steht und $X'^{\ominus}$ die Bedeutung von $X^{\ominus}$ - ausgenommen Chlorid -hat, umsetzt.

**Patentansprüche für den folgenden Vertragsstaat: AT**

1. Verfahren zur Herstellung von optisch aktiven Iridiumkomplexen der Formel (I)

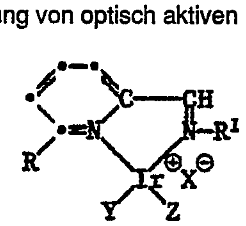

(I),

worin

R Methyl und $R^1$ ein Kohlenwasserstoffrest mit mindestens einem chiralen C-Atom oder mindestens ein Heteroatom enthaltender Kohlenwasserstoffrest mit mindestens einem chiralen C-Atom bedeuten; oder

R ein Wasserstoffatom und $R^1$ 1,2-Diphenylethyl oder ein 5- oder 6-gliedriger cycloaliphatischer oder cycloheteroaliphatischer Rest mit mindestens zwei chiralen C-Atomen und einer Phenylgruppe oder Alkylgruppe in α-Stellung bedeuten,

$X^{\ominus}$ das Anion einer einbasischen anorganischen oder organischen Säure ist, und

Y und Z, je Ethylen oder Y und Z zusammen ein offenkettiges oder cyclisches Dien mit 6 bis 10 C-Atomen, dessen Diengruppen über 1 oder 2 C-Atome verbunden sind, bedeuten, dadurch gekennzeichnet, dass man einen Diiridiumkomplex der Formel $[Ir(YZ)Cl]_2$ mit einem Pyridinaldimin der Formel (III)

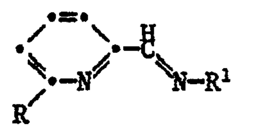

(III)

umsetzt, wobei R, $R^1$, Y und Z die zuvor angegebenen Bedeutungen haben, und anschliessend das gebildete Chlorid der Formel (I) zur Herstellung anderer Salze mit einem Salz $M^{\oplus}X'^{\ominus}$, worin $M^{\oplus}$ für ein Alkalimetall steht und $X'^{\ominus}$ die Bedeutung von $X^{\ominus}$ - ausgenommen Chlorid -hat, umsetzt.

2. Verfahren gemäss Anspruch 1, worin in Formel (I) $X^{\ominus}$ für Halogenid, $BF_4^{\ominus}$, $ClO_4^{\ominus}$, $CF_3SO_3^{\ominus}$ oder $PF_6^{\ominus}$ steht.

3. Verfahren gemäss Anspruch 2, worin $X^{\ominus}$ als Halogenid, Chlorid, Bromid oder Jodid ist.

4. Verfahren gemäss Anspruch 1, worin in Formel (I) Y und Z je für Ethylen oder Y und Z zusammen für 1,5-Cycloactadien, Norbornadien oder 1,5-Hexadien stehen.

5. Verfahren gemäss Anspruch 1, worin in Formel (I) R für Methyl steht und $R^1$ ein Rest der Formel (II)

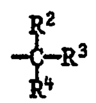

(II)

ist, worin $R^2$, $R^3$ und $R^4$ voneinander verschieden sind, wenn sie nicht mindestens 1 chirales C-Atom enthalten, und für ein Wasserstoffatom, $C_1$-$C_4$-Alkyl $C_1$-$C_4$-Alkoxy, unsubstituiertes oder durch $C_1$-$C_4$-Alkyl oder Phenyl substituiertes Cycloalkyl mit 5 bis 7 Ring-C-Atomen, unsubstituiertes oder durch $C_1$-$C_4$-Alkyl oder Phenyl substituiertes Cycloalkylalkyl mit 5 bis 7 Ring-C-Atomen und 1 oder 2 C-Atomen in der Alkylengruppe, Phenyl, Naphthyl, Benzyl oder β-Phenylethyl sind oder $R^3$ und $R^4$ zusammen durch $C_1$-$C_4$-Alkyl oder Phenyl substituiertes lineares $C_4$-oder $C_5$-Alkylen, $C_3$-oder $C_4$-Oxaalkylen oder $C_3$-Dioxaalkylen mit mindestens einem chiralen C-Atom bedeuten.

6. Verfahren gemäss Anspruch 5, worin in Formel (II) $R^2$ H bedeutet.

7. Verfahren gemäss Anspruch 5, worin in Formel (II) $R^2$ H bedeutet, $R^3$ für $C_1$-$C_4$-Alkyl, $C_1$-$C_4$-Alkoxy oder Phenyl und $R_4$ für Phenyl, Benzyl oder Naphthyl stehen, wobei $R_3$ und $R_4$ nicht beide Phenyl sind; oder $R^2$ und $R^4$ je H bedeuten und $R^3$ der Formel

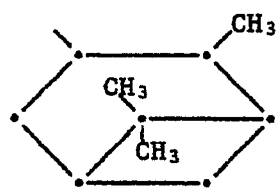

entspricht; oder die Gruppe $-CR^2R^3R^4$ der Formel

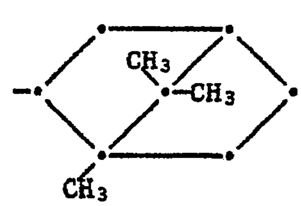

entspricht; oder $R^2$ für H steht und $R^3$ und $R^4$ zusammen in 2-Stellung durch $C_1$-$C_4$-Alkyl substituiertes Pentamethylen oder in 1- und/oder 3-Stellung durch $C_1$-$C_4$-Alkyl oder Phenyl substituiertes 2,4-Dioxapentylen bedeuten.

8. Verfahren gemäss Anspruch 1, worin R in Formel (I) für ein Wasserstoffatom steht und $R^1$ 1,2-Diphenylethyl ist oder für einen Rest der Formeln

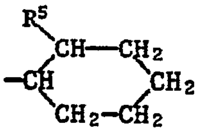 oder 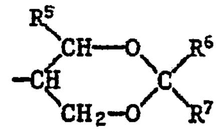

steht, worin $R^5$ $C_1$-$C_4$-Alkyl, Benzyl oder Phenyl bedeuten und $R^6$ und $R^7$ unabhängig voneinander für H, $C_1$-$C_4$-Alkyl oder Phenyl stehen.

9. Verfahren gemäss Anspruch 8, worin $R^5$ Phenyl und $R^6$ und $R^7$ Methyl oder $R^6$ H und $R^7$ Phenyl bedeuten.

10. Verwendung von optisch aktiven Iridiumkomplexen der Formel (I) gemäss Anspruch 1 als enantioselektive homogene Katalysatoren für die Transferhydrierung von prochiralen Ketonen mit sekundären Alkoholen.

**Claims for the Contracting States: BE, CH, DE, FR, GB, IT, LI, NL, SE**

1. An optically active iridium complex of the formula (I)

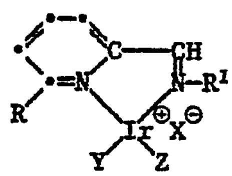 (I)

in which R is methyl and $R^1$ is a hydrocarbon radical having at least one chiral C atom or a hydrocarbon radical containing at least one hetero atom and having at least one chiral C atom; or R is a hydrogen atom and $R^1$ is 1,2-diphenylethyl or a 5-membered or 6-membered cycloaliphatic or cycloheteroaliphatic radical having at least two chiral C atoms and a phenyl group or alkyl group in the α-position, $X^\ominus$ is an anion of a monobasic inorganic or organic acid and Y and Z are each ethylene, or Y and Z together represent an open-chain or cyclic diene which has 6 to 10 C atoms and whose diene groups are bonded via 1 or 2 C atoms.

2. An iridium complex of the formula (I) according to claim 1, wherein $X^\ominus$ is halide, $BF_4^\ominus$, $ClO_4^\ominus$, $CF_3SO_3^\ominus$ or $PF_6^\ominus$.

3. An iridium complex according to claim 2, wherein $X^\ominus$, as halide, is chloride, bromide or iodide.

4. An iridium complex of the formula (I) according to claim 1, wherein Y and Z are each ethylene, or Y and Z together are 1,5-cyclooctadiene, norbornadiene or 1,5-hexadiene.

5. An iridium complex of the formula (I) according to claim 1, wherein R is methyl and $R^1$ is a radical of the formula (II)

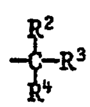 (II)

in which $R^2$, $R^3$ and $R^4$ differ from one another if they do not contain at least 1 chiral C atom, and are a hydrogen atom, $C_1$–$C_4$alkyl, $C_1$–$C_4$alkoxy, cycloalkyl having 5 to 7 ring C atoms which is unsubstituted or substituted by $C_1$–$C_4$alkyl or phenyl, cycloalkylalkyl which has 5 to 7 ring C atoms and 1 or 2 C atoms in the alkylene group and is unsubstituted or substituted by $C_1$–$C_4$alkyl or phenyl, or are phenyl, naphthyl, benzyl or β-phenylethyl, or $R^3$ and $R^4$ together are $C_1$–$C_4$alkyl-substituted or phenyl-substituted linear $C_4$- or $C_5$-alkylene, $C_3$- or $C_4$-oxaalkylene or $C_3$-dioxaalkylene having at least 1 chiral C atom.

6. An iridium complex of the formula (I) according to claim 5, wherein $R^2$ is H.

7. An iridium complex of the formula (I) according to claim 5, wherein $R^2$ is H, $R^3$ is $C_1$–$C_4$alkyl, $C_1$–$C_4$alkoxy or phenyl and $R^4$ is phenyl, benzyl or naphthyl, and $R^3$ and $R^4$ are not both phenyl; or $R^2$ and $R^4$ are each H, and $R^3$ corresponds to the formula

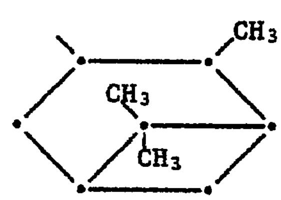

or the group $-CR^2R^3R^4$ corresponds to the formula

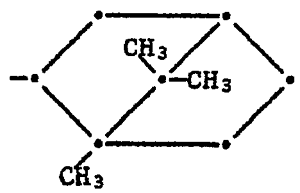

or $R^2$ is H and $R^3$ and $R^4$ are pentamethylene which is substituted in the 2-position by $C_1$–$C_4$alkyl or 2,4-dioxapentylene which is substituted in the 1- and/or 3-position by $C_1$–$C_4$alkyl or phenyl.

8. An iridum complex of the formula (I) according to claim 1, wherein R is a hydrogen atom and $R^1$ is 1,2-diphenylethyl or a radical of the formulae

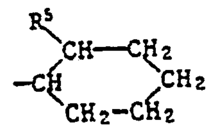

or

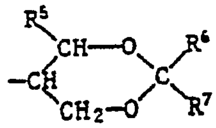

in which $R^5$ is $C_1$–$C_4$alkyl, benzyl or phenyl and $R^6$ and $R^7$ independently of one another are H, $C^1$–$C^4$alkyl or phenyl.

9. An iridium complex of the formula (I) according to claim 8, wherein $R^5$ is phenyl and $R^6$ and $R^7$ are methyl, or $R^6$ is H and $R^7$ is phenyl.

10. Use of an optically active iridium complex of the formula (I) according to claim 1 as an enantioselective homogeneous catalyst for the transfer hydrogenation of prochiral ketones with secondary alcohols.

11. A process for the preparation of an optically active iridium complex of the formula (I) according to claim 1, wherein a diiridium complex of the formula $[Ir(YZ)Cl]_2$ is reacted with a pyridinaldimine of the formula (III)

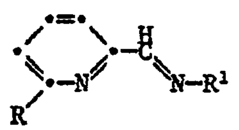

(III)

in which R,$R^1$, Y and Z have the meaning given in claim 1, and the resulting chloride of the formula (I) is then reacted with a salt $M^{\oplus}X'^{\ominus}$, in which $M^{\oplus}$ is an alkali metal and $X^{\ominus}$ has the same meaning as $X'^{\ominus}$– with the exception of chloride, in order to prepare other salts.

**Claims for the Contracting State: AT**

1. A process for the preparation of an optically active iridium complex of the formula (I)

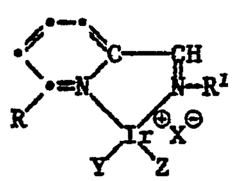

(I)

in which R is methyl and $R^1$ is a hydrocarbon radical having at least one chiral C atom or a hydrocarbon radical containing at least one hetero atom and having at least one chiral C atom; or R is a hydrogen atom and $R^1$ is 1,2-diphenylethyl or a 5-membered or 6-membered cycloaliphatic or cycloheteroaliphatic radical having at least two chiral C atoms and a phenyl group or alkyl group in the $\alpha$-position, $X^{\ominus}$ is an anion of a monobasic inorganic or organic acid and Y and Z are each ethylene, or Y and Z together represent an open-chain or cyclic diene which has 6 to 10 C atoms and whose diene groups are bonded via 1 or 2 C atoms, wherein a diiridium complex of the formula $[Ir(YZ)Cl]_2$ is reacted with a pyridinaldimine of the formula (III)

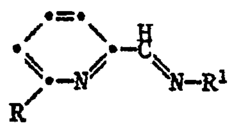

(III)

in which R,$R^1$, Y and Z have the meaning given above and the resulting chloride of the formula (I) is then reacted with a salt $M^{\oplus}X'^{\ominus}$, in which $M^{\oplus}$ is an alkali metal and $X'^{\ominus}$ has the same meaning as $X^{\ominus}$ – with the exception of chloride, in order to prepare other salts.

2. A process according to claim 1, wherein in formula (I) $X^{\ominus}$ is halide, $BF_4^{\ominus}$, $ClO_4^{\ominus}$, $CF_3SO_3^{\ominus}$ or $PF_6^{\ominus}$.

3. A process according to claim 2, wherein $X^{\ominus}$, as halide, is chloride, bromide or iodide.

4. A process according to claim 1, wherein in formula (I) Y and Z are each ethylene, or Y and Z together are 1,5-cyclooctadiene, norbornadiene or 1,5-hexadiene.

5. A process according to claim 1, wherein in formula (I) R is methyl and $R^1$ is a radical of the formula (II)

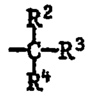

(II)

in which $R^2$, $R^3$ and $R^4$ differ from one another if they do not contain at least 1 chiral C atom, and are a hydrogen atom, $C_1$–$C_4$alkyl, $C_1$–$C_4$alkoxy, cycloalkyl having 5 to 7 ring C atoms which is unsubstituted or substituted by $C_1$–$C_4$alkyl or phenyl, cycloalkylalkyl which has 5 to 7 ring C atoms and 1 or 2 C atoms in the alkylene group and is unsubstituted or substituted by $C_1$–$C_4$alkyl or phenyl, or are phenyl, naphthyl, benzyl or β-phenylethyl, or $R^3$ and $R^4$ together are $C_1$–$C_4$alkyl-substituted or phenyl-substituted linear $C_4$- or $C_5$-alkylene, $C_3$- or $C_4$-oxaalkylene or $C_3$-dioxaalkylene having at least 1 chiral C atom.

6. A process according to claim 5, wherein in formula (II) $R^2$ is H.

7. A process according to claim 5, wherein in formula (II) $R^2$ is H, $R^3$ is $C_1$–$C_4$alkyl, $C_1$–$C_4$alkoxy or phenyl and $R^4$ is phenyl, benzyl or naphthyl, and $R^3$ and $R^4$ are not both phenyl; or $R^2$ and $R^4$ are each H, and $R^3$ corresponds to the formula

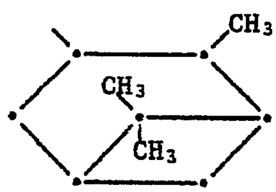

or the group $-CR^2R^3R^4$ corresponds to the formula

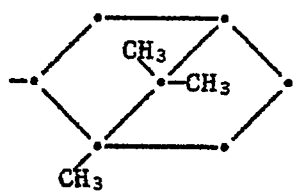

or $R^2$ is H and $R^3$ and $R^4$ are pentamethylene which is substituted in the 2-position by $C_1$–$C_4$alkyl or 2,4-dioxapentylene which is substituted in the 1- and/or 3-position by $C_1$–$C_4$alkyl or phenyl.

8. A process according to claim 1, wherein in formula (I) R is a hydrogen atom and $R^1$ is 1,2-diphenylethyl or a radical of the formula

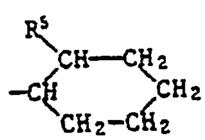 or 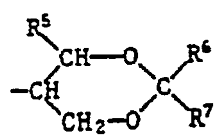

in which $R^5$ is $C_1$–$C_4$alkyl, benzyl or phenyl and $R^6$ and $R^7$ independently of one another are H, $C_1$–$C_4$alkyl or phenyl.

9. A process according to claim 8, wherein $R^5$ is phenyl and $R^6$ and $R^7$ are methyl, or $R^6$ is H and $R^7$ is phenyl.

10. Use of an optically active iridium complex of the formula (I) according to claim 1 as an enantioselective homogeneous catalyst for the transfer hydrogenation of prochiral ketones with secondary alcohols.

**Revendications pour les Etats Contractants: BE, CH, DE, FR, GB, IT, LI, NL, SE**

1. Complexes optiquement actifs de l'iridium, de formule (I)

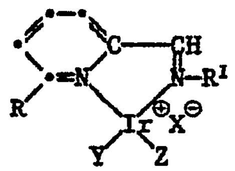
(I),

dans laquelle
R représente un groupe méthyle et $R^1$ un reste d'hydrocarbure comportant au moins un atome de C chiral ou un reste d'hydrocarbure contenant au moins un hétéro-atome avec au moins un atome de C chiral; ou bien R représente un atome d'hydrogène et $R^1$ représente un reste diphényl-2 éthyle ou un reste cycloaliphatique ou cyclohétéroaliphatique pentagonal ou hexagonal comportant au moins deux atomes de C chiral et un groupe phényle ou un groupe alkyle en position alpha,
$X^-$ représente l'anion d'un mono-acide minéral ou organique, et
Y et Z représentent chacun un groupe éthylène ou bien Y et Z forment ensemble un diène en chaîne ouverte ou cyclique comportant 6 à 10 atomes de carbone, dont les groupes diènes sont reliés par l'intermédiaire d'1 ou 2 atomes de carbone.

2. Complexes d'iridium de formule (I) selon la revendication 1, dans lesquels $X^-$ représente un ion halogénure, $BF_4^-$, $ClO_4^-$, $CF_3SO_3^-$, ou $PF_6^-$.

3. Complexes d'iridium selon la revendication 2, dans lesquels $X^-$ représente, comme halogénure, un ion chlorure, bromure ou iodure.

4. Complexes d'iridium de formule (I) selon la revendication 1, dans lesquels Y et Z représentent chacun un groupe éthylène ou bien Y et Z forment ensemble un groupe cyclooctadiène-1,5, norbornadiène ou hexadiène-1,5.

5. Complexes d'iridium de formule (I) selon la revendication 1, dans lesquels R représente un groupe méthyle et $R^1$ représente un reste de formule (II)

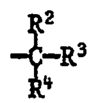
(II)

dans laquelle $R^2$, $R^3$ et $R^4$ diffèrent l'un de l'autre, quand ils ne contiennent pas au moins un atome de C chiral, et représentent chacun un atome d'hydrogène, un groupe alkyle en $C_1$ à $C_4$, alcoxy en $C_1$ à $C_4$, cycloalkyle comportant 5 à 7 atomes de C cycliques, qui n'est pas substitué ou qui est substitué par un groupe alkyle en $C_1$ à $C_4$ ou par un groupe phényle, un reste cycloalkylalkyle comportant 5 à 7 atomes de carbone de noyau et 1 ou 2 atomes de C dans le groupe alkylène (reste non substitué ou substitué par un groupe alkyle en $C_1$ à $C_4$ ou par un groupe phényle), un reste phényle, naphtyle, benzyle ou bêta-phényléthyle, ou bien $R^3$ et $R^4$ forment ensemble un reste alkylène linéaire en $C_4$ ou $C_5$, oxaalkylène en $C_3$ ou $C_4$ ou dioxaalkylène en $C_3$, comportant au moins un atome de C chiral et substitué par un groupe alkyle en $C_1$ à $C_4$ ou par un groupe phényle.

6. Complexes d'iridium de formule (I) selon la revendication 5, dans lesquels $R^2$ représente H.

7. Complexes d'iridium de formule (I) selon la revendication 5, dans lesquels $R^2$ représente H, $R^3$ représente un reste alkyle en $C_1$ à $C_4$, alcoxy en $C_1$ à $C_4$ ou phényle, et $R^4$ représente un reste phyényle, benzyle ou naphtyle, $R^3$ et $R^4$ pouvant tous deux représenter simultanément un reste phényle, ou bien $R^2$ et $R^4$ représentent chacun H et $R^3$ répond à la formule

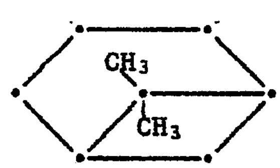

ou bien le groupe $-CR^2R^3R^4$ répond à la formule

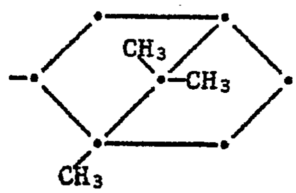

ou bien $R^2$ représente H et $R^3$ et $R^4$ forment ensemble un reste pentaméthylène, substitué en position 2 par un groupe alkyle en $C_1$ à $C_4$, ou bien un reste dioxa-2,4 pentylène, substitué en position 1 et/ou 3 par un groupe alkyle en $C_1$ à $C_4$ ou par un groupe phényle.

8. Complexes d'iridium de formule (I) selon la revendication 1, dans lesquels R représente un atome d'hydrogène et $R^1$ représente un reste diphényl-1,2 éthyle ou un reste répondant aux formules

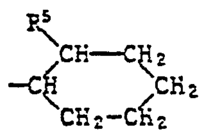

ou

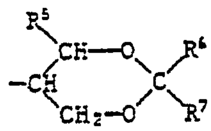

dans lesquels $R^5$ représente un reste alkyle en $C_1$ à $C_4$, benzyle ou phényle, et $R^6$ et $R^7$ représentent, indépendamment l'un de l'autre, H, un groupe alkyle en $C_1$ à $C_4$ ou un groupe phényle.

9. Complexes d'iridium de formule (I) selon la revendication 8, dans lesquels $R^5$ représente un reste phényle et $R^6$ et $R^7$ représentent chacun un groupe méthyle ou bien $R^6$ représente H et $R^7$ représente un reste phényle.

10. Utilisation de complexes optiquement actifs d'iridium, de formule (I) selon la revendication 1, comme catalyseurs homogènes énantio-sélectifs pour l'hydrogénation de transfert de cétones prochirales avec des alcools secondaires.

11. Procédé pour préparer des complexes optiquement actifs d'iridium, de formule (I) selon la revendication 1, caractérisé en ce qu'on fait réagir un complexe de diiridium de formule $[IR(YZ)Cl]_2$ avec une pyridinaldimine de formule (III)

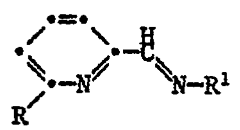

(III)

dans laquelle R, $R^1$, Y et Z ont le sens indiqué à la revendication 1, puis l'on fait réagir le chlorure ainsi formé, de formule (I) pour préparer d'autres sels, avec un sel $M^+X'^{\ominus}$, dans lequel $M^+$ représente un métal alcalin et $X'^-$a le sens de $X^-$, sauf qu'il ne représente pas un chlorure.

**Revendications pour l'Etat Contractant: AT**

1. Procédé de préparation de complexes optiquement actifs d'iridium, de formule (I)

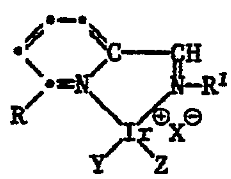

(I),

dans laquelle

R représente un groupe méthyle et $R^1$ représente un reste d'hydrocarbure comportant au moins un atome de C chiral ou un reste d'hydrocarbure contenant au moins un hétéro-atome avec au moins un atome de C chiral; ou bien R représente un atome d'hydrogène et $R^1$ représente un reste diphényl-1,2 éthyle ou un reste cycloaliphatique ou cyclohétéroaliphatique pentagonal ou hexagonal comportant au moins deux atomes de C chiral et un groupe phényle ou un groupe alkyle en position alpha,

$X^-$ représente l'anion d'un mono-acide minéral ou organique, et

Y et Z représentent chacun un groupe éthylène ou bien Y et Z forment ensemble un diène en chaîne ouverte ou cyclique comportant 6 à 10 atomes de carbone, dont les groupes diènes sont reliés par l'intermédiaire d'un ou deux atomes de carbone, procédé caractérisé en ce qu'on fait réagir un complexe de diiridium de formule $[Ir(YZ)Cl]_2$ avec une pyridinaldimine de formule (III)

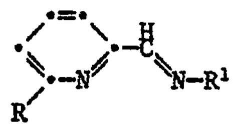

(III)

dans laquelle R, $R^1$, Y et Z ont les sens indiqués ci-dessus, puis l'on fait réagir le chlorure ainsi formé, de formule (I) pour préparer d'autres sels, avec un sel $M^+X'^-$, dans lequel $M^+$ représente un métal alcalin et $X'^-$ a le sens de $X^-$, à l'exception d'un chlorure.

2. Procédé selon la revendication 1, dans lequel, dans la formule (I), $X^-$ représente un halogénure, $BF_4^-$, $ClO_4^-$, $CF_3SO_3^-$ ou $PF_6^-$.

3. Procédé selon la revendication 2 dans lequel, $X^-$, représentant un halogénure, représente un chlorure, bromure ou iodure.

4. Procédé selon la revendication 1, dans lequel, dans la formule (I), Y et Z représentent chacun un groupe éthylène ou bien Y et Z pris ensemble représentent un reste cyclooctadiène-1,5, norbornadiène ou hexadiène-1,5.

5. Procédé selon la revendication 1, dans lequel, dans la formule (I), R représente un groupe méthyle et $R^1$ représente un reste de formule (II)

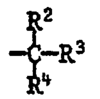

(II)

dans laquelle $R^2$, $R^3$ et $R^4$ diffèrent l'un de l'autre, lorsqu'ils ne contiennent pas au moins un atome de C chiral, et ils représentent chacun un atome d'hydrogène, un reste alkyle en $C_1$ à $C_4$, alcoxy en $C_1$ à $C_4$, cycloalkyle (non substitué ou substitué par un groupe alkyle en $C_1$ à $C_4$ ou par un groupe phényle) comportant 5 à 7 atomes de C cyclique, un reste cycloalkylalkyle (non substitué ou substitué par un groupe alkyle en $C_1$ à $C_4$ ou par un groupe phényle) comportant 5 à 7 atomes de C de noyau et 1 ou 2 atomes de C dans le groupe alkylène, un reste phényle, naphtyle, benzyle ou bêta-phényléthyle, ou bien $R^3$ et $R^4$ forment ensemble un reste alkylène en $C_4$ ou $C_5$, oxaalkylène en $C_3$ ou $C_4$ ou dioxaalkylène en $C_3$, linéaire, substitué par un groupe alkyle en $C_1$ à $C_4$ ou par un groupe phényle et comportant au moins un atome de C chiral.

6. Procédé selon la revendication 5, dans lequel, dans la formule (II), $R^2$ représente H.

7. Procédé selon la revendication 5, dans lequel, dans la formule (II), $R^2$ représente H, $R^3$ représente un reste alkyle en $C_1$ à $C_4$, alcoxy en $C_1$ à $C_4$ ou phényle, et $R^4$ représente un reste phyényle, benzyle ou naphtyle, les symboles $R^3$ et $R^4$ ne pouvant tous deux représenter simultanément un reste phényle, ou bien $R^2$ et $R^4$ représentent chacun H et $R^3$ répond à la formule

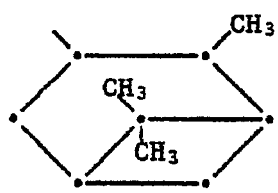

ou bien le groupe $-CR^2R^3R^4$ répond à la formule

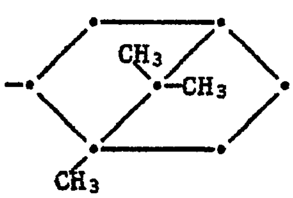

ou bien $R^2$ représente H et $R^3$ et $R^4$ forment ensemble un reste pentaméthylène, substitué en position 2 par un groupe alkyle en $C_1$ à $C_4$, ou un reste dioxa-2,4 pentylène, substitué en position 1 et/ou 3 par un groupe alkyle en $C_1$ à $C_4$ ou par un groupe phényle.

8. Procédé selon la revendication 1, dans lequel, dans la formule (I), R représente un atome d'hydrogène et $R^1$ représente un reste diphényl-1,2 éthyle ou un reste répondant aux formules

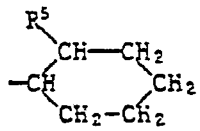

ou

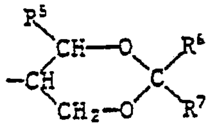

dans lesquelles $R^5$ représente un reste alkyle en $C_1$ à $C_4$, benzyle ou phényle, et $R^6$ et $R^7$ représentent, indépendamment l'un de l'autre, H, un reste alkyle en $C_1$ à $C_4$ ou un reste phényle.

9. Procédé selon la revendication 8, dans lequel $R^5$ représente un reste phényle et $R^6$ et $R^7$ représentent chacun un reste méthyle ou bien $R^6$ représente H et $R^7$ représente un reste phényle.

10. Utilisation de complexes optiquement actifs d'iridium, de formule (I) selon la revendication 1, comme catalyseurs homogènes énantio-sélectifs pour l'hydrogénation de transfert de cétones prochirales avec des alcools secondaires.